(19) Europäisches Patentamt / European Patent Office / Office européen des brevets

(11) **EP 2 056 933 B1**

(12) **EUROPÄISCHE PATENTSCHRIFT**

(45) Veröffentlichungstag und Bekanntmachung des Hinweises auf die Patenterteilung:
**27.10.2010 Patentblatt 2010/43**

(51) Int Cl.:
***A61N 2/00*** *(2006.01)*

(21) Anmeldenummer: **07819963.5**

(22) Anmeldetag: **24.08.2007**

(86) Internationale Anmeldenummer:
**PCT/EP2007/058803**

(87) Internationale Veröffentlichungsnummer:
**WO 2008/025731 (06.03.2008 Gazette 2008/10)**

(54) **VORRICHTUNG ZUR ERZEUGUNG EINES PULSIERENDEN ELEKTROMAGNETISCHEN FELDES MIT IMPULSSTEUERUNG**

DEVICE FOR GENERATING A PULSED ELECTROMAGNETIC FIELD WITH PULSE CONTROL

DISPOSITIF DE PRODUCTION D'UN CHAMP ELECTROMAGNETIQUE PULSE AU MOYEN D'UNE COMMANDE D'IMPULSIONS

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **28.08.2006 DE 102006041365**

(43) Veröffentlichungstag der Anmeldung:
**13.05.2009 Patentblatt 2009/20**

(73) Patentinhaber: **Gleim, Peter**
**9495 Triesen (LI)**

(72) Erfinder:
• **GLEIM, Peter**
**9495 Triesen (LI)**

• **KLOPP, Rainer**
**16348 Wandlitz**
**OT Stolzenhagen (DE)**

(74) Vertreter: **Gulde Hengelhaupt Ziebig & Schneider**
**Patentanwälte - Rechtsanwälte**
**Wallstrasse 58/59**
**10179 Berlin (DE)**

(56) Entgegenhaltungen:
**EP-A- 0 995 463      GB-A- 2 295 093**
**US-A- 5 800 835      US-B1- 6 440 059**

**Beschreibung**

Die Erfindung betrifft eine Vorrichtung zur Erzeugung eines pulsierenden elektromagnetischen Feldes mit Impulssteuerung.

Aus der EP 0 594 655 B1 ist eine Vorrichtung, bestehend aus Generator und Sender, bekannt, mit der ein Ionentransport aus intrakorporalen Elektrolyt-Flüssigkeiten in und durch Gefäßwände erfolgen soll und bei der die angewandten gepulsten Ströme bestimmte Eigenschaften haben, insbesondere die Amplitude der Grundpulse einer e-Funktion entsprechen und eine Pulsfolgeserie für 0,3-1 Sekunden mit Pausen von 0,7-5 Sekunden gesendet wird.

Die EP 0 995 463 B1 beansprucht eine Vorrichtung zur Beeinflussung biologischer Abläufe in einem lebenden Gewebe, bei der Impulse auf das Gewebe abgegeben werden, bei denen die Amplitude jedes Einzelimpulses einer mathematischen Beziehung entspricht mit der e-Funktion $e^{\sin(x^b)}$ worin x der Zeitverlauf und b die Anzahl der überlagerten Impulse ist.

Die mit den bekannten Vorrichtungen erzeugten Signalformen, die immer e-Funktionen entsprechen, sollen entweder Muskelbildung und Gelenkregeneration verbessern oder Stoffwechselvorgänge anregen.

Aus Rosenspire et al., Biophys. I. Vol 88, May 2005, S. 3334-3347 ist der Einfluss schwach gepulster Magnetfelder auf die Oszillation von NAD(P)H, des Redoxpotentials von Flavoprotein und der Reduktion von ROS und NO in Neutrophilen bekannt.

In JP 03-277381 wird die Vasomotionsanpassung durch Änderung der elektrischen Spannung oder Frequenz eines niederfrequenten Impulses durch Änderung einer Kondensatorspannung beschrieben. Der Erfindung liegt die Aufgabe zugrunde, eine Vorrichtung bereitzustellen, mit der durch bestimmte Impulse eines pulsierenden Magnetfeldes andere Merkmale von Körperfunktionen verbessert werden können.

Erfindungsgemäß bereitgestellt wird eine Vorrichtung zur Erzeugung eines pulsierenden elektromagnetischen Feldes mit Impulssteuerung, umfassend einen Impulsgenerator, der mit einer Spule zur Erzeugung eines elektromagnetischen Feldes verbunden ist, wobei die von dem Impulsgenerator ausgehenden Impulse periodische Impulse darstellen, die an- und absteigende Hüllkurven aufweisen mit harmonischem oder anharmonischem Schwingungsverlauf innerhalb der Hüllkurven, die Impulsfolge im Bereich von 1 Impuls pro 20 Minuten bis 4 Impulse pro Minute liegt und der Steuerung von Impulsfolge, Impulsbreite, Funktionstyp des Impulses und elektromagnetischer Flussdichte Messdaten zugrunde liegen, die mittels nichtinvasiver vitalmikroskopischer, spektrometrischer, Laser Doppler- oder Sauerstoffpartialdruck-Messverfahren aus einem Target-Gewebe Merkmale der Mikrozirkulation des Blutes wiedergeben, wobei für den Funktionstyp solche Impulse mit Exponentialfunktionen ausgenommen sind.

Es wurde gefunden, dass Impulse mit e-Funktionen und in der beschriebenen Häufigkeit der Impulsfolge von maximal 5 Sekunden bzw. maximal 3 Sekunden Pause sich zwar in einem gewissen Ausmaß auf einige Merkmale des Funktionszustandes der Mikrozirkulation sowohl beim Gesunden als auch im Krankheitsfall auswirken, jedoch nur mit kurzzeitiger und begrenzter Beeinflussung der Mechanismen der lokalen Regelung der Mikrozirkulation, so dass damit keine längerfristigen prophylaktisch und therapeutisch relevanten Veränderungen hervorgerufen werden können.

Es wurde weiterhin gefunden, dass bei einer insgesamt deutlichen Verlangsamung der Impulsfolge und bei Impulsen mit nicht exponentiell ansteigenden und absteigenden Hüllkurven infolge einer deutlich stärkeren direkten Beeinflussung der lokalen Regelmechanismen der Mikrozirkulation signifikant deutlich größere und auch länger anhaltende Merkmaländerungen des Funktionszustandes der Mikrozirkulation erzielt werden können.

Die Mikrozirkulation, die Strömung von Blutzellen und Blutplasma in den kleinsten Blutgefäßen (mit Durchmessern < 200 μm), ist der funktionell wichtigste Teil des menschlichen Blutkreislaufes, da hier der Stoffaustausch mit den Zellen eines Organgewebes realisiert wird. Dies betrifft den Antransport von Sauerstoff und Substrat zu den Zellen und den Abtransport von Stoffwechselendprodukten. Der jeweilige Funktionszustand der Mikrozirkulation eines Organs bestimmt die Regelbreite für eine Anpassung der Mikroperfusion an sich ändernde Stoffwechselbedürfnisse und somit die Organfunktion. Des Weiteren ist eine ungestörte Mikrozirkulation Vorraussetzung für einen ungehinderten Ablauf der ersten Schritte einer immunologischen Reaktion. Aufgrund dessen ist die Mikrozirkulation bereits seit einiger Zeit in das Zentrum klinisch-pathophysiologischer Forschungen gerückt. Von besonderem Interesse ist hierbei die Erforschung möglicher Beeinflussungen der lokalen Regelung der Mikrozirkulation, insbesondere der autorhythmischen Kontraktionsbewegungen der glatten Gefäßwandmuskulatur in arteriolären und venulären Mikrogefäßen (sog. Vasomotion).

Zu den wichtigsten Kriterien zur Charakterisierung einer normalen oder gestörten Mikrozirkulation gehören

- der jeweilige Verteilungszustand des Blutes in den Mikrogefäß-Netzwerken;
- die autorhythmischen (spontanen) Gefäßwandbewegungen in Arteriolen und Venolen (Vasomotion);
- der Strömungsfluss im arteriolären Zustrom und venulären Abstrom der kapillaren Netzwerke;
- rheologische Merkmale (lokaler Hämatokrit);
- Strömungsgeschwindigkeiten der Blutzellen;
- Durchmesser der Mikrogefäße;
- Möglichkeit einer Anreicherung weißer Blutzellen im Bereich der Mikrozirkulation, Adhäsionen am Endothel und Transmigrationen weißer Blutzellen ins Gewebe.

Der vasomotorische Funktionszustand determiniert wesentlich die Anpassungsbreite der Mikrozirkulation an sich ändernde Stoffwechselbedürfnisse und somit die lokale Regelbreite der Mikrozirkulation.

Erfindungsgemäß werden daher Messdaten aus der Mikrozirkulation des Blutes für die Steuerung von Impulsen eines elektromagnetischen Feldes herangezogen. Diese Messdaten sind ausgewählt aus der Gruppe, bestehend aus der venolenseitigen Sauerstoffausschöpfung, der Anzahl der blutzellperfundierten Knotenpunkte, dem venulären Strömungsfluss, dem lokalen Hämatokrit in einem Mikrogefäß, dem lokalen Hämatokrit in allen Mikrogefäßen, der spontanen arteriolären Vasomotion, dem venulären Vasomotionszustand, der Anzahl adhärierender weißer Blutzellen an einer definierten Venoleninnenwand, der lokalen Konzentrationsänderungen von Substanzen im Gewebe. Vorteilhaft ist es, mehrere dieser Merkmale heranzuziehen.

Die venolenseitige Sauerstoffausschöpfung $\Delta pO_2$ wird als prozentuale Änderung im Vergleich mit dem jeweiligen Ausgangswert zum Zeitpunkt t=0 angegeben. Bestimmt wird die absolute Differenz der Sauerstoffsättigung des Hämoglobins in den zuführenden Arteriolen und abführenden Venolen des Netzwerkes eines ausgewählten Gewebe-Targets. Als Target werden Gewebeabschnitte von Haut oder Darm, die über die angestrebten Blutgefäßnetze des Organismus verfügen und ferner zu den immologisch aktiven Organen zählen, und die weiterhin für nichtinvasive Messungen leicht zugänglich sind, ausgewählt.

Bei der Anzahl der aktuell blutzellperfundierten Knotenpunkte im definierten mikrovaskulären Netzwerk, nNP, wird die Anzahl der blutzellperfundierten Verzweigungsorte in diesem Netzwerk als Maß für den Verteilungszustand des Blutes herangezogen. Als Grenzsträmungsgeschwindigkeit der roten Blutzellen wird $V_{RBC}$=80 $\mu$m/s definiert. Die Auswertung erfolgt in + oder - (verglichen mit dem definierten Ausgangswert n=60). Grenzfälle werden mit +0,5 oder -0,5 bewertet.

Der venuläre Strömungsfluss Qven und der arterioläre Strömungsfluss Qart ist der Teilchenstrom (Blutzell-Strom) in definierten Venolen bzw. Arteriolen. Er wird in $\mu$m³/s angegeben.

Der lokale Hämatokrit in einem Blutgefäß, der auch als Tube Hämatokrit bezeichnet wird, $Hk_t$, ist der Hämatokrit in einem bestimmten Mikrogefäß. Er wird angegeben als prozentuale Änderung im Vergleich mit dem Ausgangswert.

Der Hämatokrit der Mikrozirkulatian $Hk_{MC}$ wird in allen Mikrogefäßen mit Durchmessern < 200 $\mu$m gemessen.

Der arterioläre (bzw. venuläre) Vasomotionszustand, $A_{VM}$, wird ermittelt, indem das Weg-Zeit-Diagramm der autorhythmischen Kontraktionsbewegungen glatter Muskelzellen der arteriolären Gefäßwand bestimmt wird (Messung der Distanz normal zur Mikrogefäß-Längsachse von Endotheloberfläche zu gegenüberliegender Endotheloberfläche zu äquidistanten Messzeitpunkten; pro Sekunde 60 Meßwerte; Ermittlung der zusammengesetzten Schwingung; FOURIER-Analyse; Ermittlung des Amplituden-Frequenz-Spektrums). Kriterium ist der Flächeninhalt A unter der Einhüllenden des Amplituden-Frequenz-Spektrums der arteriolären Vasomotion (eine zusammengesetzte Schwingung). Angegeben wird der Wert als prozentuale Veränderung im Vergleich mit den Ausgangswerten.

Die Anzahl der adhärierenden weißen Blutzellen an einer definierten Venoleninnenwand, nWBC/A, wird an der definierten Innenfläche der Venole mit A=18000 $\mu$m² gemessen. Gezählt werden alle weißen Blutzellen, die länger als 20 Sekunden am Endothel anhaften.

Lokale Konzentrationsänderungen verschiedener Substanzen im Gewebe werden z.B. für Mediatoren, das Adhäsionsmolekül ICAM-1 und andere gemessen. Sie werden in relativen Einheiten angegeben, von 0-10, wobei 0 kein Nachweis bedeutet und der Wert 10 dem höchsten in einer Stichprobe ermittelten Wert zugeordnet wird.

Grundlagen für die Messung dieser Merkmale an Humangewebe sind z.B. beschrieben in Bollinger et al., Microvasc Res 7(1974)61-72; Fagrell B, Angiology 23(1972)284-298; Fagrell et al., Am J Physiol 233(1977)H318-321; Wiedemann et al., An introduction to microcirculation, Academic Press, NY 1981; und Lankowicz JR (Ed.): Topics in Fluorescence Spectroscopy, Plenum Press, New York, London, Vol. 1-5 (1991-1997), auf die hier Bezug genommen wird.

Für alle erhobenen Messdaten wird auf ein parameterfreies Prüfverfahren für kleine Stichproben zurückgegriffen. Zur Anwendung gelangt der WILCOXON-Rangsummentest auf dem Signifikanzniveau $\alpha$=5%. Die kritischen Werte für T werden der Literatur entnommen (Ferguson G, Statistical analysis in psychology and education, McGraw-Hill, NY 1959, 318).

Von besonderer Bedeutung ist die Vasomotion. Prophylaktisch und therapeutisch relevant ist eine Beeinflussung der gestörten Vasomotion hin zu einem physiologischen Vasomotionsrhythmus, d.h. das "Aufprägen" eines physiologischen Vasomotionsrhythmus im Krankheitsfall. Im Krankheitsfall sind die Vasomotionsbewegungen arteriolärer und venulärer Mikrogefäße erheblich verändert (meist deutlich verlangsamt; bisweilen nur weniger als 1 bis 2 Vasomotionsbewegungen in Verlauf mehrerer Minuten). Erfindungsgemäß wird angestrebt, die gestörte Vasomotion in den Bereich der physiologischen Vasomotion zu führen (etwa 1-10, insbesondere 1-4 Vasomotionsbewegungen pro Minute). Entsprechendes gilt für pathologische Zustände mit erheblich beschleunigter Vasomotion.

Es wurde daher gefunden, dass neben den beschriebenen Wirkungen bestimmter pulsierender elektromagnetischer Felder im Gewebe vor allem eine Rhythmusänderung der gestörten spontanen arteriolären Vasomotion erreicht werden kann, zumeist im Sinne einer Stimulierung der autorhythmischen Kontraktionsbewegungen der glatten Gefäßwandmuskulatur in kleinsten Blutgefäßen (Vasomotion von Arteriolen und Venolen). Bei deutlichen Änderungen der Impulsfolge (Verlangsamung der Impulsfolge gegenüber bekannten Verfahren) und bei Impulsen mit nicht exponentiell ansteigenden und/oder absteigenden oder abrupt abfallenden Hüllkurven können signifikant deutlich größere Merkmaländerungen der Mikrozirkulation des Blutes bewirkt werden.

In deutlicher Abweichung zu bekannten Methoden, bei denen gegebenenfalls Blutdruck, Atemfrequenz oder Herzfrequenz (Makrozirkulation) für eine Magnetfeldbehandlung berücksichtigt werden und zwar insbesondere während der Behandlung selbst, werden mit der Vorrichtung der Erfindung die bereits insbesondere vor der Behandlung gemessenen oben genannten speziellen Werte der Mikrozirkulation für die Behandlung mit äußerst geringen Frequenzen zugrundegelegt.

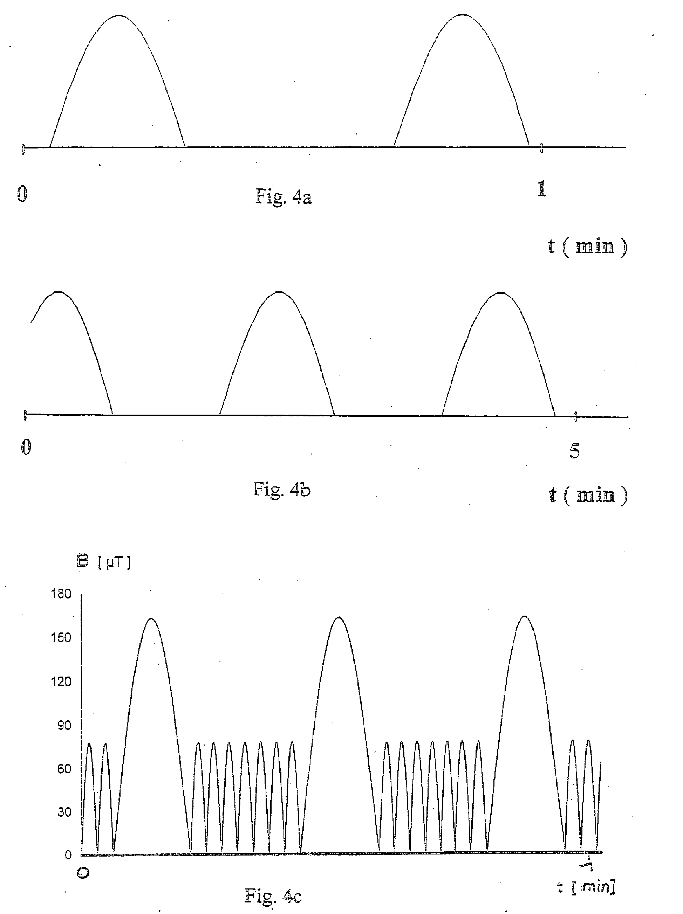

Bevorzugt werden daher von der erfindungsgemäßen Vorrichtung Impulsfolgen erzeugt, die im Bereich von 1 Impuls/10 Minuten bis 2 Impulse/1 Minute liegen, vorzugsweise von 1 Impuls/5 Minuten bis 3 Impulse/1 Minute, insbesondere von 1 Impuls/2 Minuten bis 1 Impuls/1 Minute. In Fig. 4a und 4b sind beispielhaft solche vorteilhaften Impulsfolgen dargestellt ohne Bezug auf die Intensität.

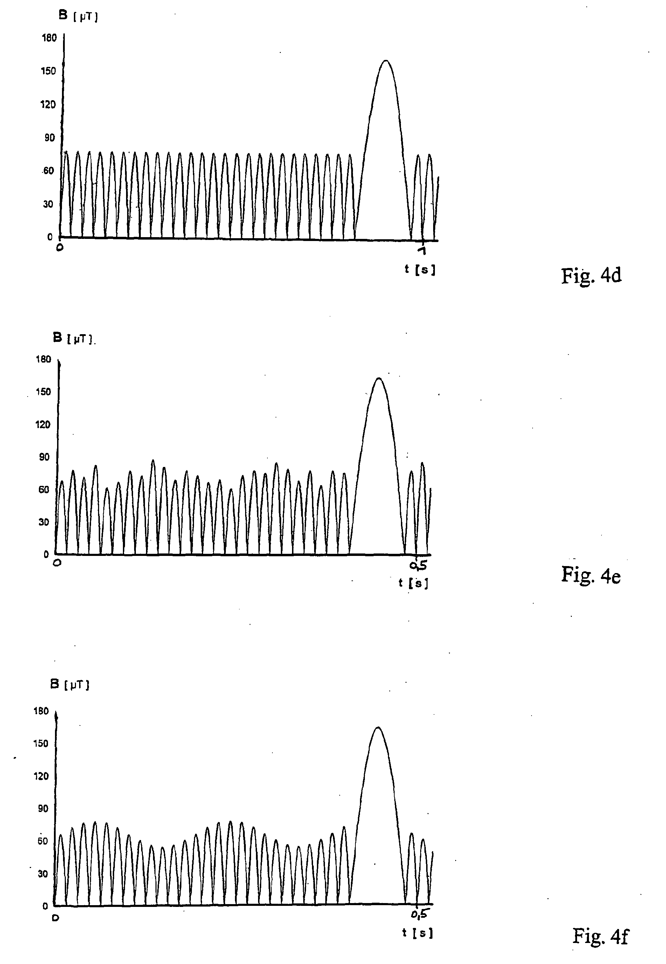

Unter "Impulsfolge" im Sinne der vorliegenden Erfindung wird verstanden der Abstand solcher Schwingungsmaxima (Impulsmaxima) voneinander, die über der Intensitäts-Nulllinie liegen in einem Intensitäts-Zeit-Diagramm wie z.B. in Fig. 4c dargestellt. Falls eine Basisschwingung vorhanden ist, die hinsichtlich der Intensität gleichbleibend, stochastisch unterschiedlich oder sinusförmig unterschiedlich sein kann, wie in Fig. 4d bzw. 4e bzw. 4f dargestellt, bedeutet "Impulsfolge" den Abstand solcher Schwingungsmaxima voneinander, der deutlich über der Basisschwingung liegt.

Impulsfolge ist damit die Häufigkeit des Auftretens maximaler Beträge der Hüllkurve in der Zeiteinheit.

So kann beispielsweise eine kontinuierliche Basisschwingung einer Intensität von 80 μT vorliegen mit einer Impulsbreite von ca. 30 μs und ein deutlich stärkerer Einzelimpuls von 150 μT mit einer größeren Impulsbreite von 0,3 s, wobei der stärkere Einzelimpuls drei bis fünf mal pro Minute auftritt. Dies entspricht dann ebenfalls der Impulsfolge im Sinne der Erfindung.

Eine solche Impulsanordnung kann vorteilhaft sein, d.h. zum o.g. Impuls bzw. zur o.g. Impulsfolge (Fig. 4a und 4b) einen höherfrequenten Impuls mit geringerer elektromagnetischer Flussdichte B hinzuzufügen. Diese zusätzlichen Impulse können in ihren Amplituden (und Frequenzen) in verschiedener Weise variieren, wie exemplarisch in den Fig. 4c bis 4h dargestellt ist. Sie liegen im Allgemeinen bei 50 bis 80 μT.

Vorteilhaft liegt die Breite eines Einzelimpulses bei 50 bis 300 ms und die Breite eines Basisimpulses bei 10 bis 60 ms; bevorzugt sind 80 bis 200 ms bzw. 20 bis 40 ms.

Die sog. "Intensität" oder Impulsstärke ist physikalisch die elektromagnetische Flussdichte B mit der Einheit Tesla.

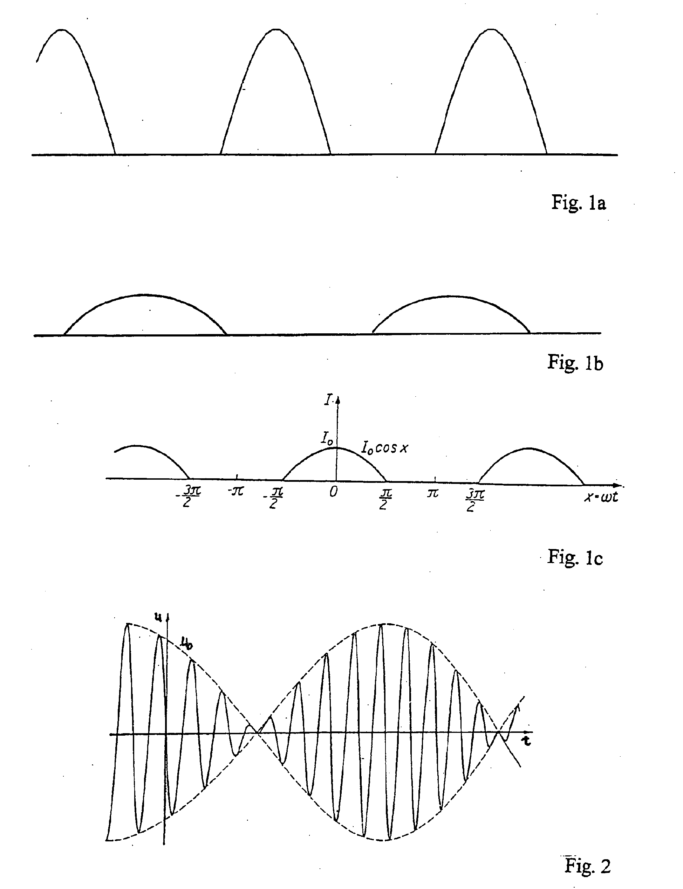

Die von der erfindungsgemäßen Vorrichtung erzeugten Impulse werden periodisch abgegeben und stellen bei zeichnerischer Darstellung in ihren Hüllkurven bogenförmige wie sinus- oder cosinusförmige bis parabelähnliche Konstrukte dar. Innerhalb der Hüllkurven treten harmonische Schwingungen mit gleicher oder unterschiedlicher Amplitude auf, die sich auch zu anharmonischen Schwingungen überlagern können. Unter "Hüllkurve" wird die Kurve verstanden, die die Maxima von unterschiedlich hohen Amplituden einer bestimmten Folge von Amplituden berührt und damit diese Folge im ansteigenden und absteigenden Teil "umhüllt" (siehe Fig. 2). Bei überlagerten anharmonischen Schwingungen betrifft dies immer nur die Maxima der benachbarten nächsthöheren Amplitude.

Vorzugsweise entsprechen somit die Impulse einem Funktionstyp, dessen An- und Abstieg der Hüllkurven etwa bogenförmig wie bei gleichgerichteten Strömen verläuft.

Die Impulse sind zusammengesetzte Schwingungen bzw. Wellen, die aus einer Vielzahl von Teilschwingungen wie harmonischen oder anharmonischen unterschiedlicher Amplitude und Frequenz gebildet werden, wobei die Teilfrequenzen von ~ 20 bis 3000 Hz liegen.

"Hüllkurve" verbindet die unterschiedlichen Amplituden der Teilschwingungen (Amplitude = max. Elongation einer Teilschwingung). Die "Hüllkurve" spiegelt näherungsweise den Verlauf der zusammengesetzten Schwingung bzw. Welle wider.

Insbesondere bevorzugt ist ein Funktionstyp des Impulses für die Hüllkurve, der dem Typ eines gleichgerichteten Kosinusstromes entspricht. Ein solcher gleichgerichteter Kosinusstrom lautet in der Entwicklung einer FOURIER-Reihendarstellung periodischer Funktionen wie folgt

$$I(x) = \frac{I_0}{\pi}\left(1 + \frac{\pi}{2}\cos x + \frac{2}{1\cdot 3}\cos 2x - \frac{2}{3\cdot 5}\cos 4x + \frac{2}{5\cdot 7}\cos 6x - +\ldots\right)$$

In graphischer Darstellung entspricht sie der Fig. 1c.

Unter "bogenförmiger Verlauf einer Kurve" wird ein solcher Kurvenverlauf verstanden, der keine Wendepunkte hat und negativ gekrümmt ist, wie er beispielhaft in Fig. 1a, 1b und 1c gezeigt ist.

Beispiele für die Überlagerung harmonischer Schwingungen und die dabei entstehende anharmonische Schwingung sind in Fig. 4 aufgeführt.

Optimale Behandlungsresultate werden erzielt, wenn das Signal (Impuls) auf Basis simultan gemessener Funktionsmerkmale der Mikrozirkulation entsprechend variiert wird. Dabei können Amplituden und Frequenzen der Einzelimpulse, Impulsfolgen bzw. Impulspausen oder Intensitäten geändert werden. Intensitäten von Nano-Tesla-Bereich bis zum Milli-Tesla-Bereich sind möglich z.B. 50 nT bis 800 mT, üblicherweise liegen sie jedoch im Mikro-Tesla-Bereich bei etwa 5-300 $\mu$T.

Sehr gute Messresultate biologischer Wirkungen hinsichtlich Vasomotion und Funktionszustand der Mikrozirkulation wurden bei Anwendung elektromagnetischer Flussdichten im Bereich von ca. 50 $\mu$T bis ca. 250 $\mu$T, vorzugsweise 80 $\mu$T bis 150 $\mu$T, erhalten. Dies sind immer gemittelte Beträge.

Für die angestrebten Wirkungen im Bereich der Mikrozirkulation ist eine Variation der elektromagnetischen Flussdichten im Vergleich mit einer Variation der Impulstypen und vor allem der Impulsfolge von geringerer Bedeutung.

Die erfindungsgemäße Vorrichtung kann bei Gesunden im Sinne einer Leistungssteigerung oder bei infekt- und/oder stressexponierten Personen angewendet werden, bei Älteren mit eingeschränkter körperlicher Leistungsfähigkeit und reduzierter Immunabwehr und auch im Krankheitsfall. Auch die Behandlung von Säugetieren fällt unter den Umfang der Erfindung. Für eine prophylaktische Anwendung sind insbesondere die Wirkungen auf die Vasomotion (Merkmal $A_{VM}$) und den Verteilungszustand des Blutes in den mikrovaskulären Netzwerken (Merkmal nNP) von Bedeutung und damit für die Steigerung der körperlichen Leistungsfähigkeit und verbesserte Organfunktionen infolge einer Erweiterung der mikrozirkulatorischen Regelbreite.

Im Vergleich mit Vorrichtungen, bei denen Impulse, die e-Funktionen folgen, und höhere Impulsfolgen (häufigeres Auftreten der Impulse in der Zeiteinheit) angewendet werden, werden mit der erfindungsgemäßen Vorrichtung deutlich größere und wesentlich länger anhaltende Merkmaländerungen des Funktionszustandes der Mikrozirkulation erzielt.

Wenn beispielsweise bei Impulsen mit e-Funktionen eine Änderung des Merkmals $A_{VM}$ von maximal 10 % erreicht wird und ein Abklingen der Änderung nach ca. 20 Minuten auftritt, zeigt das erfindungsgemäße Signal ein Maximum von ca. 22 %, bleibt einen bestimmten Zeitraum nur leicht abfallend auf dieser Höhe und klingt erst langsam bei ca. 50 bis 60 Minuten ab. Ingesamt wird dadurch eine deutlich verbesserte Gesamtwirkung erzielt.

Vorteilhaft ist ein (komplementär-therapeutischer) Einsatz der erfindungsgemäßen Vorrichtung bei verschiedenen Erkrankungen, wie z.B. periphere Durchblutungsstörungen, diabetische Mikroangiopathie, diabetische Polyneuropathie, Wundheilungs- und Knochenheilungsstörungen sowie Ulcerationen (wie beispielsweise bei Ulcus cruris im Rahmen einer chronischen Veneninsuffizienz), bei multimorbiden geriatrisch Patienten u.a.

Die Erfindung soll nachstehend durch Beispiele näher erläutert werden. In den dazu gehörigen Zeichnungen zeigen:

Fig. 1a     Bogenförmiger Impuls mit steilem Anstieg und steilem Abfall;

Fig. 1b     Bogenförmiger Impuls mit flachem Anstieg und fla- chem Abfall;

Fig. 1c     Impulstyp gleichgerichteter Kosinusstrom;

Fig. 2     Hüllkurve (gestrichelte Linie) einer amplitudenmodifizierten Welle;

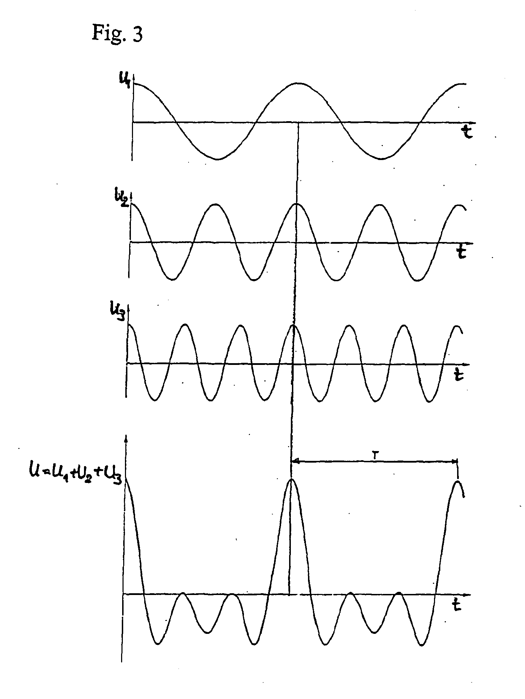

Fig. 3     Überlagerung von drei harmonischen Schwingungen $U_1$, $U_2$ und $U_3$ gleicher Amplitude und Phase und dem Frequenzverhältnis $\omega_1$, $\omega_2$ und $\omega_3$ = 1:2:3;

Fig. 4a     bevorzugte Impulsfolge 2 pro 1 Minute;

Fig. 4b     Impulsfolge 1 pro 3 Minuten;

Fig. 4c     Intensitäts-Zeit-Diagramm mit Impulsfolge 3 pro Minute (150 ms und 163 $\mu$T) mit Basisimpulsen in kürzerer Folge und mit niedrigerer Intensität (30 ms und 78 $\mu$T) ;

Fig. 4d     Ausschnitt aus einer Impulsfolge 1 Impuls pro Minute (150 ms und 163 $\mu$T) mit Basisimpulsen (30 ms und 78 $\mu$T) kontinuierlich;

Fig. 4e     Ausschnitt aus einer Impulsfolge 1 Impuls pro Minute mit Basisimpulsen in stochastischem Inten- sitätsverlauf und

Fig. 4f    Ausschnitt aus einer Impulsfolge 1 Impuls pro Minute mit Basisimpulsen in sinusförmigem Inten- sitätsverlauf.

In den Figuren sind die Impulsbreiten, die nur im Bereich von Millisekunden liegen, nicht maßstäblich dargestellt.

Beispiel 1

Bei einer Reihe von Probanden mit peripheren Durchblutungsstörungen werden mittels einer vitalmikroskopischen Untersuchungseinheit, mittels Reflexionsspektroskopie, Laser-Mikrofluss-Messung und Weißlicht-Spektroskopie repräsentive Merkmale des Funktionszustandes der Mikrozirkulation gemessen: Anzahl der blutzellperfundierten Knotenpunkte in einem definierten mikrovaskulären Netzwerk nNP, arterioläre Vasomotion/Flächeninhalt unter den Einhüllenden des Amplituden-Frequenzspektrums der arteriolären Vasomotion $A_{VM}$.
Danach erfolgt der Einsatz eines Impulsgeneratos, mit dem Impulse erzeugt werden, die einer elektromagnetischen Spule zugeführt werden. Die Spule steht in Kontakt mit einer Hautfläche (Targetgewebe). Es werden mittels dieser Vorrichtung Impulse auf das Targetgewebe im Abstand von 1 Tag, 3 Tagen, 6 Tagen, 9 Tagen und 12 Tagen gegeben. Die Messung der o.g. Parameter erfolgte jeweils 10 Minuten nach Beendigung der Impulsgabe.

Anzahl der Probanden: 16
Alter: 55-65 Jahre
Impulsfolge: 5 pro Minute
Impulstyp: näherungsweise gleichgerichtet sinusförmig
Impulsintensität: Einzelimpuls mit 180 $\mu$T und einer Impulsbreite von 150 ms; zusätzlich Basisimpuls mit 60 $\mu$T und einer Impulsbreite von 30 ms
Behandlungsdauer: 2 x 25 Minuten im Abstand von 2 h
Behandlungsfolge: jeden 2. Tag

Die statistische Auswertung erfolgte mit dem Wilcoxon-Rangsummentest, $\alpha$=5%.
Die prozentuale Änderung für $A_{VM}$ betrug bereits am 3. Tag etwa 11% und erhöhte sich zum 12. Tag auf ca. 22 %.
Die prozentuale Änderung für nNP betrug bereits am 3. Tag etwa 10 % und erhöhte sich zum 12. Tag auf ca. 24 %.

Vergleichsbeispiel 1

Es wurde in gleicher Weise wie im Beispiel 1 mit einer Gruppe von 16 Probanden gearbeitet.

Impulsfolge: 30 pro Sekunde
Impulstyp: spezielle Exponentialfunktion $e^{sin(x\ hoch\ 3)}$ gemäß EP 995463
Impulsintensität: 50 $\mu$T mit 30 ms Impulsbreite

Die statistische Auswertung erfolgte mit dem Wilcoxon-Rangsummentest, $\alpha$=5%.
Die prozentuale Änderung für $A_{VM}$ betrug am 3. Tag etwa 3% und erhöhte sich zum 12. Tag auf ca. 4 %.
Die prozentuale Änderung für nNP betrug am 3. Tag etwa 4 % und erhöhte sich zum 12. Tag auf ca. 6 %.
Diese Änderungen stellen keine therapeutisch relevanten Änderungen dar und zeigen, dass sowohl Impulstyp als auch Impulsfolge keine wesentliche Beeinflussung des lokalen Regelmechanismus der Mikrozirkulation mit sich bringen.

Beispiel 2

Bei einer Reihe von Probanden mit diabetische Mikroangiopathie wurde in gleicher Weise wie im Beispiel 1 gearbeitet.

Anzahl der Probanden: 14
Alter: 60-70 Jahre

Die statistische Auswertung erfolgte mit dem Wilcoxon-Rangsummentest, $\alpha$=5%.
Die prozentuale Änderung für $A_{VM}$ betrug bereits am 3. Tag mehr als 9 % und erhöhte sich zum 12. Tag auf ca. 25 %.
Die prozentuale Änderung für nNP betrug bereits am 3. Tag etwa 12 % und erhöhte sich zum 12. Tag auf ca. 30 %.

Vergleichsbeispiel 2

Es wurde in gleicher Weise wie im Beispiel 2 mit einer Gruppe von 14 Probanden gearbeitet. Die statistische Auswertung erfolgte mit dem Wilcoxon-Rangsummentest, $\alpha$=5%.

Die prozentuale Änderung für $A_{VM}$ betrug am 3, Tag etwa 5% und erhöhte sich zum 12. Tag auf ca. 8 %.

Die prozentuale Änderung für nNP betrug am 3. Tag ebenfalls etwa 5 % und erhöhte sich zum 12. Tag auf ca. 7 %.

Diese Änderungen stellen keine therapeutisch relevanten Änderungen dar und zeigen, dass sowohl Impulstyp als auch Impulsfolge keine wesentliche Beeinflussung des lokalen Regelmechanismus der Mikrozirkulation mit sich bringen.

Beispiel 3

Bei einer Reihe von gesunden, älteren Probanden wurde in gleicher Weise wie im Beispiel 1 gearbeitet.

Anzahl der Probanden: 16
Alter: 55-65 Jahre, ohne pathologischen Befund

Die statistische Auswertung erfolgte mit dem Wilcoxon-Rangsummentest, $\alpha$=5%.

Die prozentuale Änderung für $A_{VM}$ betrug bereits am 3. Tag etwa 7 % und erhöhte sich zum 12. Tag auf ca. 12 %.

Die prozentuale Änderung für nNP betrug bereits am 3. Tag etwa 8 % und erhöhte sich zum 12. Tag auf ca. 16 %.

Vergleichsbeispiel 3

Es wurde in gleicher Weise wie im Beispiel 3 mit einer Gruppe von 16 Probanden gearbeitet. Die statistische Auswertung erfolgte mit dem Wilcoxon-Rangsummentest, $\alpha$=5%.

Die prozentuale Änderung für $A_{VM}$ betrug am 3. Tag etwa 4 % und erhöhte sich zum 12. Tag auf ca. 5 %.

Die prozentuale Änderung für nNP betrug am 3. Tag etwa 5 % und erhöhte sich zum 12. Tag auf ca. 6 %.

Diese Änderungen zeigen, dass bei Anwendungen sowohl dieses Impulstyps als auch Impulsfolge im Vergleich zur Anwendung der erfindungsgemäßen Vorrichtung nur eine geringe Beeinflussung des Funktionszustandes der Mikrozirkulation mit sich bringen. Die Werte im Beispiel 3 zeigen am Ende des Untersuchungszeitraumes dagegen eine 2- bis 3-fache Steigerung.

Die prozentuale Änderung für $A_{VM}$ betrug bereits am 3. Tag etwa 7 % und erhöhte sich zum 12. Tag auf ca. 12 %.

Die prozentuale Änderung für nNP betrug bereits am 3. Tag etwa 8 % und erhöhte sich zum 12. Tag auf ca. 16 %.

**Patentansprüche**

1. Vorrichtung zur Erzeugung eines pulsierenden elektromagnetischen Feldes mit Impulssteuerung, umfassend einen Impulsgenerator, der mit einer Spule zur Erzeugung eines elektromagnetischen Feldes verbunden ist, wobei die von dem Impulsgenerator ausgehenden Impulse periodische Impulse darstellen, die an- und absteigende Hüllkurven aufweisen mit harmonischem oder anharmonischem Schwingungsverlauf innerhalb der Hüllkurven; die Impulsfolge im Bereich von 1 Impuls/20 Minuten bis 4 Impulse/1 Minute und die Impulsstärke im Bereich von 5 - 300 $\mu$T liegt und der Steuerung von Impulsfolge, Impulsbreite, Funktionstyp des Impulses und elektromagnetischer Flussdichte Messdaten zugrunde liegen, die mittels nichtinvasiver vitalmikroskopischer, spektrometrischer, Laser-Doppler- oder Sauerstoffpartialdruck-Messverfahren aus einem Target-Gewebe Merkmale der Mikrozirkulation des Blutes wiedergeben, wobei für den Funktionstyp solche Impulse mit Exponentialfunktionen ausgenommen sind.

2. Vorrichtung nach Anspruch 1, **dadurch gekennzeichnet, dass** die Impulse einem Funktionstyp entsprechen, dessen An- und Abstieg der Hüllkurven bogenförmig verläuft.

3. Vorrichtung nach Anspruch 2, **dadurch gekennzeichnet, dass** der Funktionstyp des Impulses für die Hüllkurve dem Typ eines gleichgerichteten Kosinusstromes entspricht.

4. Vorrichtung nach Anspruch 1, **dadurch gekennzeichnet, dass** die Impulsfolge im Bereich von 1 Impuls/5 Minuten bis 3 Impulse/1 Minute liegt, vorzugsweise von 1 Impuls/2 Minuten bis 1 Impuls/1 Minute.

5. Vorrichtung nach Anspruch 1, **dadurch gekennzeichnet, dass** der Impulssteuerung Messdaten aus der Mikrozirkulation des Blutes zugrunde liegen, ausgewählt aus der Gruppe, bestehend aus der venolenseitigen Sauerstoffausschöpfung, der Anzahl der blutzellperfundierten Knotenpunkte, dem venulären Strömungsfluss, dem lokalen Hämatokrit in einem Mikrogefäß, dem lokalen Hämatokrit in allen Mikrogefäßen, dem spontanen arteriolären Vasomotionszustand, der Anzahl adhärierender weißer Blutzellen an einer definierten Venoleninnenwand, der lokalen Konzentrationsänderungen von Substanzen im Gewebe und mehreren dieser Merkmale.

6. Vorrichtung nach Anspruch 4, **dadurch gekennzeichnet, dass** der Impulssteuerung Messdaten für die venolenseitige Sauerstoffausschöpfung zugrunde liegen.

7. Vorrichtung nach Anspruch 4, **dadurch gekennzeichnet, dass** der Impulssteuerung Messdaten für den spontanen arteriolären Vasomotionszustand zugrunde liegen.

8. Vorrichtung nach Anspruch 1, **dadurch gekennzeichnet, dass** die Impulsstärke im Bereich von 5 bis 250 $\mu$T liegt, vorzugsweise 50 bis 250 $\mu$T, insbesondere 80 bis 150 $\mu$T.

**Claims**

1. A device for generating a pulsed electromagnetic field with pulse control, said device comprising a pulse generator connected to a coil for generating an electromagnetic field, wherein
the pulses provided by the pulse generator represent periodic pulses having ascending and descending envelope curves with harmonic or inharmonic oscillation profiles within the envelope curves;
the pulse sequence is in the range of from 1 pulse/20 minutes to 4 pulses/1 minute and the pulse strength is in the range of 5 - 300 $\mu$T; and the control of pulse sequence, pulse width, pulse function type and electromagnetic flux density is based on measured data which, obtained using non-invasive vital-microscopic, spectrometric, laser-Doppler or oxygen partial pressure measuring methods on a target tissue, represent features of the blood microcirculation,
with exponential functions as pulse function type being excluded.

2. The device according to claim 1, **characterized in that** the pulses correspond to a type of function where the ascent and descent of the envelope curves have an arc-shaped profile.

3. The device according to claim 2, **characterized in that** the pulse function type for the envelope curve corresponds to the type of a rectified cosine current.

4. The device according to claim 1, **characterized in that** the pulse sequence is in the range of from 1 pulse/5 minutes to 3 pulses/1 minute, preferably from 1 pulse/2 minutes to 1 pulse/1 minute.

5. The device according to claim 1, **characterized in that** the pulse control is based on measured data from the blood microcirculation selected from the group consisting of oxygen depletion at the venule side, number of blood cell-perfused nodal points, venular stream flow, local hematocrit in a microvessel, local hematocrit in all microvessels, state of spontaneous arteriolar vasomotion, number of adhering white blood cells on a defined venule inner wall, local changes of substance concentrations in tissue and a plurality of the above features.

6. The device according to claim 4, **characterized in that** the pulse control is based on measured data for oxygen depletion at the venule side.

7. The device according to claim 4, **characterized in that** the pulse control is based on measured data for the state of spontaneous arteriolar vasomotion.

8. The device according to claim 1, **characterized in that** the pulse strength is in the range of from 5 to 250 $\mu$T, preferably from 50 to 250 $\mu$T, especially from 80 to 150 $\mu$T.

**Revendications**

1. Dispositif de production d'un champ électromagnétique pulsé avec commande d'impulsions, comportant un générateur d'impulsions qui est relié à une bobine afin de générer un champ électromagnétique,

les impulsions sortant du générateur d'impulsions représentant des impulsions périodiques qui ont des courbes d'enveloppe ascendantes et descendantes ayant une allure d'oscillations harmonique et non harmonique à l'intérieur des courbes d'enveloppe ;

la séquence d'impulsions étant située dans la plage de 1 impulsion/20 minutes à 4 impulsions/1 minute et l'intensité des impulsions étant dans la plage de 5 à 300 $\mu$T, et

la commande de la séquence d'impulsions, de la largeur des impulsions, du type de fonction des impulsions et de la densité du flux électromagnétique étant basée sur des données de mesure qui reproduisent des caractéristiques de la microcirculation du sang au moyen de procédés de mesure non effractifs, capillaroscopiques, spectrométriques ou de procédés de mesure à laser à effet Doppler ou à pression partielle d'oxygène à partir d'un tissu cible, le type de fonction ne comprenant pas des impulsions ayant des fonctions exponentielles.

2. Dispositif selon la revendication 1, **caractérisé en ce que** les impulsions correspondent à un type de fonction dont les flancs ascendants et descendants des courbes d'enveloppe ont une forme d'arc.

3. Dispositif selon la revendication 2, **caractérisé en ce que** le type de fonction des impulsions pour la courbe d'enveloppe correspond au type d'un courant sinusoïdal redressé.

4. Dispositif selon la revendication 1, **caractérisé en ce que** la séquence d'impulsions est située dans la plage de 1 impulsion/5 minutes à 3 impulsions/1 minute, de préférence de 1 impulsion/2 minutes à 1 impulsion/1 minute.

5. Dispositif selon la revendication 1, **caractérisé en ce que** la commande d'impulsions est basée sur des données de mesure provenant de la microcirculation du sang, sélectionnées par le groupe composé de l'extraction d'oxygène dans les veinules, le nombre des points nodaux de perfusion de globules sanguins, du flux de circulation dans les veines, l'hématocrite local dans un microvaisseau, l'hématocrite local dans tous les microvaisseaux, de l'état artériolaire spontané de modification du calibre vasculaire, du nombre de globules blancs adhérents à une paroi interne définie de veinule, des modifications locales de concentration de substances dans le tissu et de plusieurs de ces caractéristiques.

6. Dispositif selon la revendication 4, **caractérisé en ce que** la commande d'impulsions est basée sur des données de mesure pour l'extraction d'oxygène dans les veinules.

7. Dispositif selon la revendication 4, **caractérisé en ce que** la commande d'impulsions est basée sur des données de mesure pour l'état artériolaire spontané de modification du calibre vasculaire.

8. Dispositif selon la revendication 1, **caractérisé en ce que** l'intensité des impulsions est située dans la plage de 5 à 250 $\mu$T, de préférence de 50 à 250 $\mu$T et plus particulièrement de 80 à 150 $\mu$T.

Fig. 1a

Fig. 1b

Fig. 1c

Fig. 2

Fig. 3

0

Fig. 4a

1

t ( min )

0

Fig. 4b

5

t ( min )

B [μT]

180

150

120

90

60

30

0

O

Fig. 4c

t [ min]

Fig. 4d

Fig. 4e

Fig. 4f

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0594655 B1
- EP 0995463 B1
- JP 3277381 A
- EP 995463 A

**In der Beschreibung aufgeführte Nicht-Patentliteratur**

- **Rosenspire et al.** *Biophys. I.,* Mai 2005, vol. 88, 3334-3347
- **Bollinger et al.** *Microvasc Res,* 1974, vol. 7, 61-72
- **Fagrell B.** *Angiology,* 1972, vol. 23, 284-298
- **Fagrell et al.** *Am J Physiol,* 1977, vol. 233, 318-321
- **Wiedemann et al.** An introduction to microcirculation. Academic Press, 1981
- Topics in Fluorescence Spectroscopy. Plenum Press, 1991, vol. 1-5
- **Ferguson G.** Statistical analysis in psychology and education. McGraw-Hill, 1959, 318